# EUROPÄISCHE PATENTANMELDUNG

(11) **EP 0 647 620 A1**
(43) Veröffentlichungstag der Anmeldung: **12.04.1995**
(21) Anmeldenummer: 94114936.1
(22) Anmeldetag: 22.09.1994
(51) Int. Cl.: C07C 255/35, C07C 253/30

(54) **2,4,5-Trihalogenzimtsäurenitrile und Verfahren zu ihrer Herstellung**

(30) Priorität: 02.10.1993 DE 4333711
(71) Anmelder: HOECHST AKTIENGESELLSCHAFT, D-65929 Frankfurt am Main (DE)
(72) Erfinder: Beller, Matthias, Dr., D-65527 Niedernhausen (DE); Fischer, Hartmut, Dr., D-65719 Hofheim (DE); Forstinger, Klaus, Dr., Baikum Thane 400 608 (IN); Pfirmann, Ralf, Dr., D-64347 Griesheim (DE); Strutz, Heinz, Dr., D-61250 Usingen (DE)

(57) **Zusammenfassung**

2,4,5-Trihalogenzimtsäurenitrile der Formel I, dadurch gekennzeichnet, daß 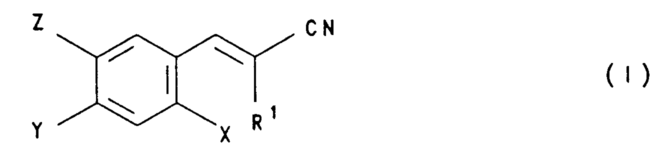
- R¹: Wasserstoff, (C₁-C₁₂)-Alkyl gerade oder verzweigt, Fluor, Chlor, Phenyl wobei der Phenylrest gegebenenfalls mit (C₁-C₄)-Alkyl oder (C₁-C₂)-Alkoxy substituiert sein kann und
- X, Y, Z: gleich oder verschieden Fluor, Chlor oder Brom bedeuten.

Ferner ein Verfahren zur Herstellung der Verbindungen der Formel I, indem man eine Verbindung der Formel (II) 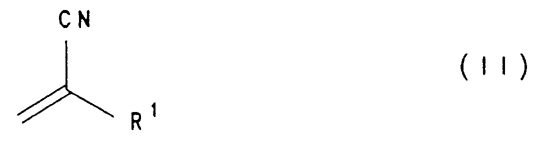
worin R¹ die oben definierte Bedeutung hat, in Gegenwart eines Palladium-Katalysators, einer Base und gegebenenfalls eines Lösungsmittels und/oder stabilisierenden Liganden und/oder Ammoniumsalzen mit einer Verbindung der Formel III 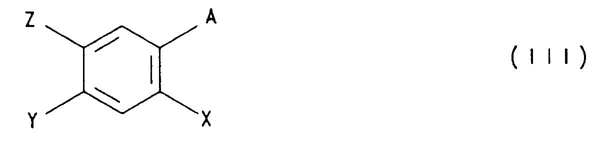
worin X, Y, Z die oben definierte Bedeutung besitzen und A Jod, Brom oder Chlor bedeutet, bei Temperaturen von 80 - 200° C umsetzt.

## Beschreibung

Die vorliegende Erfindung betrifft halogenierte Zimtsäurenitrile sowie Verfahren zu ihrer Herstellung.

Die genannten Verbindungen haben technische Bedeutung als Zwischenprodukte zur Herstellung von Wirkstoffen für Pharmaka, Herbizide und Fungizide.

Die neu hergestellten Verbindungen sind auf klassischem Weg über die Zimtsäureester oder die Zimtaldehyde nur sehr schwer herzustellen, weil dafür als eigentliche Ausgangsstoffe die entsprechenden Trihalogenbenzaldehyde erforderlich sind (vgl. dazu Houben-Weyl, Band E5 S. 362, 1348).

Als Synthesevariante bietet sich deshalb die Arylierung des Acrylnitrils an. Diese durch Palladiumverbindungen katalysierte Reaktion ist seit den Arbeiten von R.F. Heck prinzipiell bekannt; Zusammenfassungen dazu finden sich in "Palladium Reagents in Organic Syntheses", Academic Press N.Y. (1985) und in Organic Reactions 27, 345-390 (1982). Als Olefinkomponenten werden bevorzugt Acrylsäureester und Olefine eingesetzt, während die Reaktion mit Acrylnitril nur in wenigen Beispielen versucht wird. So beschreiben R.F. Heck et al. in J. Org. Chem. 43, 2945 (1978) Syntheseversuche von p-Amino-zimtsäurenitril aus Bromanilin oder Iodanilin, wobei nur aus letzterem eine Ausbeute von 53 % erreicht wurde, während die Bromverbindung kein Zielprodukt lieferte. In J. Organomet. Chem. 258 (1983), 101 wird die Herstellung von p-Formyl-Zimtsäurenitril beschrieben, aber auch darauf hingewiesen, daß die Reaktivität von Arylbromiden, die keine konjugationsfähigen Substituenten enthalten, begrenzt ist.

Wegen der allgemeinen Bedeutung und der vielseitigen Verwendbarkeit dieser Stoffklasse stellt es eine lohnende Aufgabe dar, neue Verbindungen aus dieser Gruppe von Stoffen bereitzustellen, um das Spektrum ihrer Anwendungsmöglichkeiten nicht nur zu ergänzen, sondern auch durch eine Nuancierung stofflicher Eigenschaften zu bereichern und zu erweitern. Zudem eröffnen sich mittels dieser neuen Verbindungen zusätzlich Wege zur Herstellung von Folgeprodukten, deren Bedeutung voranstehend bereits erwähnt wurde.

Diese Aufgabe wird gelöst durch 2,4,5-Trihalogenzimtsäurenitrile der Formel I, dadurch gekennzeichnet, daß 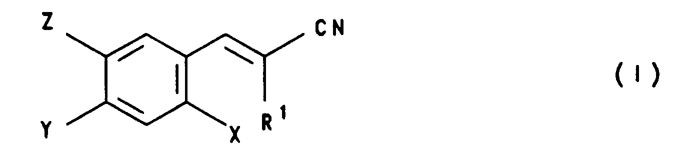
- R¹: Wasserstoff, (C₁-C₁₂)-Alkyl gerade oder verzweigt, Fluor, Chlor, Phenyl, wobei der Phenylrest gegebenenfalls mit (C₁-C₄)-Alkyl oder (C₁-C₂)-Alkoxy substituiert sein kann und
- X, Y, Z: gleich oder verschieden Fluor, Chlor oder Brom bedeuten.

Eine gewisse Bedeutung kommt den Verbindungen der Formel (I) zu, worin R¹ für Wasserstoff oder Methyl und X, Y, Z für Fluor oder Chlor stehen.

Interessant sind auch die Verbindungen der Formel (I) worin X, Y, Z Fluor bedeutet, insbesondere für R¹ gleich Wasserstoff oder Methyl.

Hervorzuheben sind auch die Verbindungen der Formel I, worin X und Y Chlor und Z Fluor oder Y und Z Fluor und X Chlor bedeuten, insbesondere für R¹ gleich Wasserstoff oder Methyl.

Diese halogenierten Zimtsäurenitrile lassen sich in vorteilhafter Weise herstellen, indem man Acrylnitril oder substituierte Acrylnitrile der Formel (II) 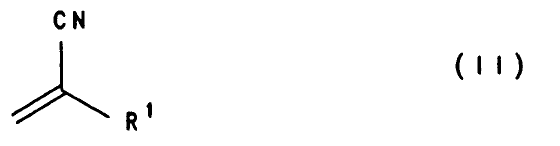
worin R¹ die bereits genannte Bedeutung hat, in Gegenwart eines Palladium-Katalysators, einer Base und gegebenenfalls eines Lösungsmittels und/oder eines stabilisierenden Liganden und/oder eines Ammoniumsalzes mit einer Verbindung der Formel III 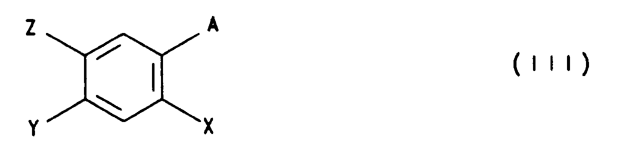
worin X, Y, Z die oben definierte Bedeutung besitzen und A Iod, Brom oder Chlor bedeutet, bei Temperaturen von 80 - 200°C umsetzt.

Allgemein einsetzbar sind, unabhängig vom Substitutionsmuster der Reste X, Y und Z diejenigen Arylhalogenide (III), in denen A ein Iodatom ist. Wenn die Reste X, Y und Z nur Fluor- oder Chloratome darstellen, so werden bevorzugt Arylbromide eingesetzt. Im Falle des bevorzugten Substitutionsmusters Y = Z = Fluor und X = Chlor kann A vorteilhaft ebenfalls Chlor sein.

Die Reaktion wird in der Regel in Lösung durchgeführt. Geeignete inerte organische Lösemittel können aliphatische oder aromatische Kohlenwasserstoffe, Chlor- oder Dichlorbenzol, Ether wie Tetrahydrofuran, Dioxan, Di-Tri- oder Tetraglyme, Anisol, Nitrile wie Acetonitril, i-Butyronitril oder Benzonitril, Alkohole wie Butanol oder 2-Ethylhexanol sein. Bevorzugt werden jedoch aprotisch-polare Lösemittel verwendet, wie Dimethyl- oder Diethylsulfoxid, N,N-Dialkylamide von aliphatischen Carbonsäure oder alkylierte Lactame. Ganz besonders geeignete Lösemittel sind N,N-Dimethylacetamid, N,N-Dimethylformamid und N-Methylpyrrolidon.

Da bei der Reaktion Halogenwasserstoff abgespalten wird, ist es vorteilhaft, diesen durch Zusatz einer Base abzufangen. Dazu geeignet sind tertiäre Amine wie Trialkylamine mit Alkylresten C₂-C₆, cyclische sekundäre Amine wie Piperidin, Piperazin oder Morpholin und die Alkali- oder Erdalkalisalze von aliphatischen oder aromatischen Carbonsäuren oder der Kohlensäure, wie Natrium, Kalium- oder Calciumacetat und Natrium- oder Kaliumcarbonat.

Die Reaktion wird sowohl homogen durch lösliche Palladiumverbindungen als auch durch heterogene Palladiumkatalysatoren beschleunigt.

Geeignete lösliche Palladiumverbindungen sind Palladiumacetat, Palladiumchlorid oder -bromid und die daraus leicht zugänglichen Tetrachloro- bzw. Tetrabromopalladate als Alkali- oder Ammoniumsalze. Sehr geeignete Katalysatorvorstufen sind auch die Komplexe des Palladiumchlorids mit Acetonitril, Benzonitril oder Triphenylphosphan, oder die Komplexe des nullwertigen Palladiums wie Tetrakis(triphenylphosphan)-palladium.

Zur Stabilisierung des aktiven Katalysators in der Reaktionslösung kann es vorteilhaft sein, Komplexbildner zuzusetzen. Geeignet dafür sind Nitrile, N-haltige Liganden, wie 2,2'-Dipyridyl oder o-Phenanthrolin oder Phosphite. Bevorzugt angewendet werden Phosphane und quartäre Ammoniumsalze, wobei diese allein oder auch in Kombination eine Stabilisierung und Aktivierung des Palladiumkatalysators bewirken.

Als einzähnige Monophosphane kommen insbesondere Triarylphosphane, Dialkylarylphosphane, Diarylphosphane, Diarylalkylphosphane und Trialkylphosphane in Frage, wobei die Alkylgruppen 1 bis 12 C-Atome enthalten und die Arylgruppen Phenyl- oder Naphthylgruppen sind, die jeweils mit C₁₋₄-Alkyl, C₁₋₃-Alkoxy oder SO₃Na substituiert sein können.

Als Beispiele seien genannt: Triphenylphosphan, Tri-o- und Tri-p-tolylphosphan, Tri(methoxyphenyl)-phosphan, Tri(m-sulfonatophenyl)-phosphan, Tricyclohexylphosphan, Triisopropylphosphan oder Di-isopropyl-phenylphosphan. Unter den einzähnigen Phosphanen werden besonders bevorzugt Triphenylphosphan, Tri-o-tolyl-phosphan und Tricyclohexylphosphan eingesetzt.

Es kann vorteilhaft sein, chelatisierende Bisphosphane allein oder im Gemisch mit den Monophosphanen einzusetzen, wobei bevorzugt das Bis(diphenylphosphino)-ethan oder -propan verwendet werden.

Einen sehr vorteilhaften Effekt auf den Ablauf der Katalysereaktion ergibt der Zusatz von Ammoniumbromiden oder -chloriden. Wegen der sehr guten Löslichkeit und ihrer hohen Stabilität in der Reaktionsmischung werden quartäre Ammoniumsalze bevorzugt, die in Mengen von 1 bis 100 Mol-%, bezogen auf das umzusetzende Arylhalogenid, zum Katalysatorsystem zugegeben werden können. Als besonders geeignet haben sich erwiesen: Tetraethyl-, Tetrabutyl-, Tetrahexyl-, Methyltrioctyl-, Benzyl-triethyl- und Benzyl-tributyl-ammoniumchlorid bzw. -bromid.

Der aktive Katalysator wird bevorzugt aus den Komponenten in der Reaktionslösung oder unmittelbar vorher in separater Lösung gebildet, ohne daß eine Isolierung oder ein kompliziertes Präparationsverfahren notwendig ist. Die Menge des eingesetzten Palladiums beträgt 0,01 bis 5 Mol-%, bevorzugt 0,2 bis 1,0 Mol-%, bezogen auf das Arylhalogenid.

Heterogene Palladiumkatalysatoren sind metallisches Palladium, Palladiumschwarz oder Palladium auf einem Trägermaterial fixiert. Als Trägermaterialien können beliebig inerte Feststoffe eingesetzt werden. Beispielhaft seien hier Aktivkohle, Aluminiumoxide, Siliciumoxide, Magnesiumoxid, Alumosilikate, Kaliumcarbonat, Bariumsulfat und Calciumcarbonat genannt. Besonders geeignete Trägermaterialien sind Aktivkohle, Aluminiumoxid, Siliciumdioxid und Titandioxid.

Die Ausführung des Verfahrens läßt eine Reihe von Varianten zu. Die einfachste besteht darin, alle Komponenten im Reaktionskolben vorzulegen und das Gemisch ausreichende Zeit auf die erforderliche Temperatur zu bringen.

Eine Verbesserung der Selektivität läßt sich im Falle von besonders polymerisationsfähigen Acrylnitril-Derivaten erreichen, wenn diese während der Reaktion nachdosiert werden. Bei Ansätzen, die deutlich größer sind als im Labor üblich, wird es aus Gründen der Sicherheit notwendig, die Reaktion so zu steuern, daß eine kontrollierte Abführung der Reaktionswärme möglich ist. Dies gelingt beispielsweise dadurch, daß eine flüssige, organische Base verwendet wird, die unter Reaktionsbedingungen, gegebenenfalls gleichzeitig mit Katalysator-Vorstufe, eindosiert wird.

Für die Reaktion sind Temperaturen von 80-200°C nötig; Arylbromide reagieren zwischen 80-160°C, bevorzugt 120-140°C, während Arylchloride Temperaturen von 120-200°C, bevorzugt 140-180°C erfordern.

Die erforderlichen Zeiten für einen hohen Umsatz bewegen sich zwischen 1 Stunde bis 16 h.

### Beispiel 1

### 2,4-Dichlor-5-fluorzimtsäurenitril

24,4 g (0,1 Mol) 1-Brom,2,4-dichlor-5-fluorbenzol, 12,7 g (0,1 Mol) Natriumcarbonat (gemahlen + getrocknet) und 10,6 g (0,2 Mol) Acrylnitril werden zusammen mit 40 ml Dimethylacetamid in einen Reaktionskolben gegeben. Davon getrennt wird eine Lösung aus 0,224 g (1,0 mMol) Palladiumacetat und 10,5 g (0,05 Mol) Tetraethylammoniumbromid in 30 ml Dimethylacetamid bereitet. Diese wird dann unter gutem Rühren zur Suspension der anderen Komponenten gegeben und auf 135°C erwärmt. Innerhalb von 3 h wird quantitativer Umsatz erreicht.

Die Reaktionslösung wird zunächst filtriert, dann in 500 ml kaltes Wasser eingegossen. Dabei scheidet sich das Zielprodukt als hellbrauner Feststoff ab, der auf einem Filter gesammelt und getrocknet wird. Das Rohprodukt wird aus Cyclohexan umkristallisiert.

Das Primärprodukt besteht aus 36 % cis-Isomer, 64 % trans-Isomer. Bei der Kristallisation entsteht eine
- Fraktion 1:: 12,7 g (56 % d. Th.)
Nach Einengen des LM kristallisiert
- Fraktion 2:: 9,0 g (41 % d. Th.).

In der Fraktion 1 ist das trans-Isomer auf 94 % angereichert.

Das Massenspektrum bestätigt für beide Isomere die nominale molare Masse (bezogen auf ³⁵Cl-Isotrop) von M = 215.

### H-NMR-Spektren:

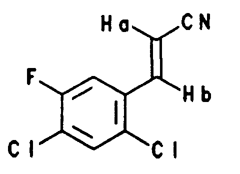

Ha = 5,78/5,95 ppm J_{ab} = 17 Hz
Hb = 7,63/7,79 ppm 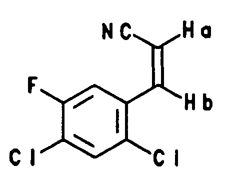
Ha = 5,60/5,73 ppm J_{ab} = 12,5 Hz
Hb = 7,85/7,96 ppm

### Beispiel 2

### 2,4-Dichlor-5-fluorzimtsäurenitril

Die Verbindung läßt sich auch mit einem phosphanstabilisierten Katalysatorsystem herstellen.
12,2 g (0.05 Mol) 1-Brom-2,4-dichlor-5-fluorbenzol, 5,0 g (0.06 Mol) Natriumacetat und 5,4 g (0,1 Mol) Acrylnitril werden zusammen mit 15 ml DMAc in einen Reaktionskolben gegeben.

110 mg (0.5 mMol) Palladium werden in 10 ml Dimethylacetamid gelöst und mit 264 mg (1,0 mMol) Triphenylphosphan und 200 mg (0,5 mMol) Bis(diphenylphosphino)-ethan (DIPHOS) versetzt. Nach kurzem Rühren bei Zimmertemperatur hat sich der Pd(II)-Phosphankomplex gebildet.

Diese Lösung wird zur Mischung der Ausgangsstoffe gegeben, und der gesamte Ansatz wird auf 140°C erwärmt.

Nach 8 h wurde ein Umsatz von 95 % erreicht. Zur Aufarbeitung wurde die Reaktionsmischung mit Methyl-t-butylether verdünnt, mit Wasser ausgeschüttelt und die organische Phase eingeengt. Das zurückbleibende Rohprodukt wird aus Cyclohexan umkristallisiert. Es wurde eine Ausbeute von 10,0 g (76 % d. Th.) erreicht.

Das Produkt ist hinsichtlich cis-/trans-Isomerenverteilung und Reinheit identisch mit dem Stoff aus Beispiel 1, wie GC und H-NMR-Spektren belegen.

### Beispiel 3

### 2,4,5-Trifluorzimtsäurenitril

10.6 g (0.05 Mol) 2,4,5-Trifluorbrombenzol, 5,0 g (0.06 Mol) Natriumacetat und 5,4 g (0,01 Mol) Acrylnitril werden zusammen mit 20 ml Dimethylacetamid in einen Reaktionskolben gegeben.

Davon getrennt wird eine Katalysatorlösung bereitet aus 224 mg (0,1 mMol) Palladiumacetat, 524 mg (0,2 mMol) Triphenylphosphan und 400 mg (0,1 mMol) Bis(diphenylphosphino)-ethan (= DIPHOS) in 30 ml Dimethylacetamid.

Nach Lösung der Katalysatorkomponenten und Ausbildung des gelben Palladiumkomplexes wird diese Lösung zum obigen Ansatz gegeben und unter guter Rührung auf 140°C erwärmt.

Es wird innerhalb von 7 h ein Umsatz von 86 % erzielt. Einziges Produkt ist das gewünschte 2,4,5-Trifluorzimtsäurenitril, das als Gemisch der cis-/trans-Isomeren im Verhältnis von ca. 40/60 anfällt.

Das Produkt wird aus der Ansatzlösung isoliert, indem zuerst mit Dichlormethan verdünnt und anschließend mit Wasser extrahiert wird. Die Lösung in CH₂Cl₂ wird konzentriert und das Konzentrat schließlich destilliert. Der Siedepunkt des Zielproduktes beträgt 65°C bei 0,1 mbar, Fp = 94-97°C.

Das Massenspektrum bestätigt für beide Isomere die molare Masse von M = 183. Das H-NMR-Spektrum bestätigt die Struktur. 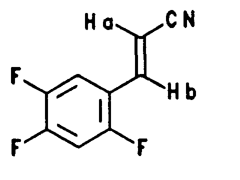
Ha = 5,86/6,04 ppm J_{ab} = 17 Hz
Hb = 7,30/7,49 ppm 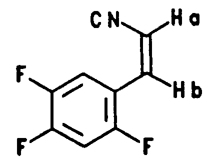
Ha = 5,54/5,66 ppm J_{ab} = 12,5 Hz
Hb = 6,95/7,09 ppm

### Beispiel 4

### 2,4-Dichlor-5-fluor-(α-methyl)-Zimtsäurenitril

24,4 g (0,1 Mol) 1-Brom-2,4-dichlor-5-fluorbenzol, 12,7 g (0,12 Mol) Natriumcarbonat und 13,4 g (0,2 Mol) Methacrylnitril werden zusammen mit 40 ml Dimethylacetamid in einen Reaktionskolben gegeben. Davon getrennt wird eine Lösung von 0,224 g (1,0 mMol) Palladiumacetat und 10,5 g (0,05 Mol) Tetraethyl-ammoniumbromid in 30 ml Dimethylacetamid bereitet.
Diese wird unter gutem Rühren zur Suspension der anderen Komponenten gegeben, und die Reaktionsmischung wird anschließend auf 135°C erwärmt. Nach 2 h wird durch GC quantitativer Umsatz nachgewiesen.

Zur Aufarbeitung wird die Reaktionsmischung mit Methyl-t-butylether verdünnt, mit Wasser ausgeschüttelt und die organische Phase destilliert. Nach Abdestillieren des Lösemittels bleibt ein Rohprodukt zurück, das im Vakuum destilliert wird, Kp₁ = 105°C.

Die isolierte Substanz besteht aus drei Isomeren, die aufgrund des Massenspektrums alle die Molmasse M = 229 (bezogen auf ³⁵Cl-Isotop) haben. Das H-NMR-Spektrum beweist folgende Strukturen:

### Isomer A, 68 %:

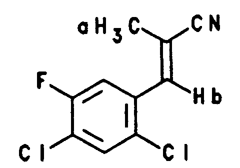

Ha = 2,20 ppm (Dublett)
Hb = 7,70/7,78 ppm

### Isomer B, 29 %:

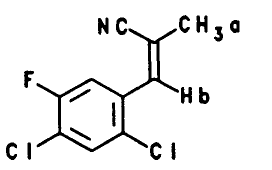

Ha = 2,04 ppm (Dublett)
Hb = 7,14/7,26 ppm

### Isomer C, 2 %:

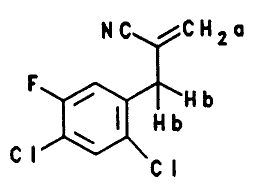

Ha = 5,74/6,0 ppm
Hb = 3,64 ppm

## Patentansprüche

1. 2,4,5-Trihalogenzimtsäurenitrile der Formel I, dadurch gekennzeichnet, daß 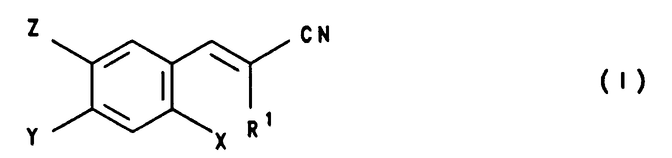
R¹ Wasserstoff, (C₁-C₁₂)-Alkyl gerade oder verzweigt, Fluor, Chlor, Phenyl wobei der Phenylrest gegebenenfalls mit (C₁-C₄)-Alkyl oder (C₁-C₂)-Alkoxy substituiert sein kann und
X, Y, Z gleich oder verschieden Fluor, Chlor oder Brom bedeuten.

2. Verbindungen nach Anspruch 1, dadurch gekennzeichnet, daß R¹ Wasserstoff oder Methyl und X, Y, Z Fluor oder Chlor bedeuten.

3. Verbindungen nach Anspruch 1 oder 2, dadurch gekennzeichnet, daß X, Y, Z Fluor bedeutet.

4. Verbindungen nach Anspruch 1 oder 2, dadurch gekennzeichnet, daß X und Y Chlor und Z Fluor bedeuten.

5. Verbindungen nach Anspruch 1 oder 2, dadurch gekennzeichnet, daß Y und Z Fluor und X Chlor bedeuten.

6. Verfahren zur Herstellung der Verbindungen nach Anspruch 1, dadurch gekennzeichnet, daß man eine Verbindung der Formel (II) 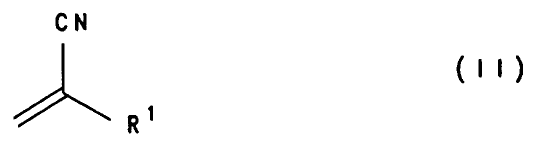 worin R¹ die oben definierte Bedeutung hat, in Gegenwart eines Palladium-Katalysators, einer Base und gegebenenfalls eines Lösungsmittels und/oder stabilisierenden Liganden und/oder Ammoniumsalzen mit einer Verbindung der Formel III 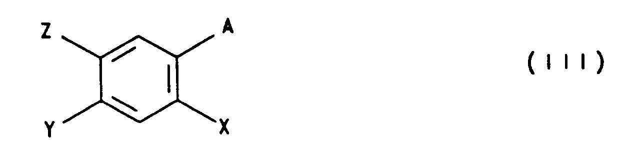 worin X, Y, Z die oben definierte Bedeutung besitzen und A Jod, Brom oder Chlor bedeutet, bei Temperaturen von 80 - 200° C umsetzt.

7. Verfahren nach Anspruch 6, dadurch gekennzeichnet, daß A Brom oder Iod, insbesondere Iod, bedeutet.

8. Verfahren nach Anspruch 6 oder 7, dadurch gekennzeichnet, daß als Lösungsmittel aliphatische Kohlenwasserstoffe, aromatische Kohlenwasserstoffe, Chlorbenzole, Dichlorbenzol, Tetrahydrofuran, Dioxan, Diglyme, Triglyme oder Tetraglyme, Anisol, Acetonitril, i-Butyronitril, Benzonitril, Butanol, 2-Ethylhexanol, insbesondere Dimethylsulfoxid, Diethylsulfoxid, N,N-Dialkylamide von aliphatischen Carbonsäuren, alkylierte Lactame, bevorzugt N,N-Dimethylacetamid, N,N-Dimethylformamid und N-Methylpyrrolidon eingesetzt werden.

9. Verfahren nach mindestens einem der Ansprüche 6 bis 8, dadurch gekennzeichnet, daß als Base tertiäre Amine und/oder Alkali- oder Erdalkalisalze von aliphatischen oder aromatischen Carbonsäuren oder der Kohlensäure, insbesondere C₂-C₆-Alkylamine, cyclische sekundäre Amine, bevorzugt Piperidin, Piperazin oder Morpholin, Natrium, Kaliumacetat, Calciumacetat und/oder Natrium- oder Kaliumcarbonat eingesetzt werden.

10. Verfahren nach mindestens einem der Ansprüche 6 bis 9, dadurch gekennzeichnet, daß als Palladium-Katalysator lösliche Palladiumverbindungen, insbesondere Palladiumacetat, Palladiumchlorid, Palladiumbromid, Tetrachloropalladat, Tetrabromopalladat, Komplexe des Palladiumdichlorids mit Acetonitril, Benzonitril, Triphenylphosphan, Tetrakis(triphenylphosphan)palladium eingesetzt werden.

11. Verfahren nach mindestens einem der Ansprüche 6 bis 9, dadurch gekennzeichnet, daß als Palladium-Katalysator ein heterogener Palladium-Katalysator, insbesondere Palladiumschwarz oder Palladium auf einem Trägermaterial eingesetzt werden.

12. Verfahren nach Anspruch 11, dadurch gekennzeichnet, daß als Trägermaterial Aktivkohle, Aluminiumoxid, Siliciumoxid, Magnesiumoxid, Titanoxide, Alumosilikate, Kaliumcarbonat, Bariumsulfat oder Calciumcarbonat, insbesondere Aktivkohle, Aluminiumoxid, Siliciumoxid oder Titandioxid eingesetzt werden.

13. Verfahren nach mindestens einem der Ansprüche 6 bis 10, dadurch gekennzeichnet, daß als stabilisierende Liganden Nitrile, Amine, Phosphane oder Phosphite, insbesondere Arylphosphane oder Alkylphosphane, bevorzugt Triphenylphosphan, Tri-o-tolylphosphan oder Tricyclohexylphosphan eingesetzt werden.

14. Verfahren nach mindestens einem der Ansprüche 6 bis 13, dadurch gekennzeichnet, daß als Ammoniumsalze quartäre Ammoniumbromide oder - chloride, insbesondere Tetraethyl-, Tetrabutyl-, Tetrahexyl-, Methyltrioctyl-, Benzyltriethyl und/oder Benzyltributylammoniumchlorid oder -bromid eingesetzt werden.

15. Verfahren nach mindestens einem der Ansprüche 1 bis 14, dadurch gekennzeichnet, daß 1 bis 100 Mol-% Ammoniumsalz, bezogen auf das Arylhalogenid, eingesetzt werden.

16. Verfahren nach mindestens einem der Ansprüche 1 bis 15, dadurch gekennzeichnet, daß 0,01 bis 5 Mol-%, insbesondere 0,2 bis 1 Mol-% Palladium-Katalysator, bezogen auf das Arylhalogenid, eingesetzt werden.
